**Europäisches Patentamt**

(19) **European Patent Office**

**Office européen des brevets**

(11) Veröffentlichungsnummer: **0 090 284**
**B1**

(12) **EUROPÄISCHE PATENTSCHRIFT**

(45) Veröffentlichungstag der Patentschrift:
**27.12.85**

(51) Int. Cl.⁴: **C 07 C 15/00**, C 07 C 1/20

(21) Anmeldenummer: **83102673.7**

(22) Anmeldetag: **18.03.83**

(54) **Verfahren zur Herstellung von Aromaten aus Methanol und/oder Dimethylether.**

(30) Priorität: **27.03.82 DE 3211398**

(43) Veröffentlichungstag der Anmeldung:
**05.10.83 Patentblatt 83/40**

(45) Bekanntmachung des Hinweises auf die Patenterteilung:
**27.12.85 Patentblatt 85/52**

(84) Benannte Vertragsstaaten:
**BE DE FR GB NL**

(56) Entgegenhaltungen:
**EP - A - 0 041 621**
**US - A - 3 928 483**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft,
Carl-Bosch-Strasse 38, D-6700 Ludwigshafen (DE)**

(72) Erfinder: **Hoelderich, Wolfgang, Dr., Mannheimer Strasse 18 C, D-6710 Frankenthal (DE)**
Erfinder: **Mross, Wolf Dieter, Dr.,
Anselm-Feuerbach-Strasse 21, D-6710 Frankenthal (DE)**
Erfinder: **Schwarzmann, Matthias, Dr.,
Carl-Bosch-Strasse 54, D-6703 Limburgerhof (DE)**

## Beschreibung

Es sind eine Reihe von Verfahren zur Herstellung von Aromaten aus Methanol und/oder Dimethylether bekannt.

In neuerer Zeit gewinnen Bemühungen, Methanol zur Herstellung von Kohlenwasserstoff zu verwenden, zunehmendes Interesse. Methanol kann man aus Kohle durch Kohlevergasung und Herstellung von Synthesegas durch Konvertierung mit Hilfe bewährter Technik herstellen. Gelingt es, Methanol in technisch einfacher Weise zu Aromaten wie Benzol, Toluol und Xylolen umzuwandeln, können die heute üblichen Weiterverarbeitungsverfahren auch bei der Verwendung von Kohle als Rohstoff beibehalten werden. In den vergangenen Jahren sind daher eine Reihe von Verfahren entwickelt worden, die die Herstellung von Aromaten aus Kohlenwasserstoffen mit Siedepunkt 205°C, aus niederen Alkoholen und/oder Ethern und aus Kohlenmonoxid und Wasserstoff in einstufiger Reaktion an Aluminosilikatzeolithen, zum Gegenstand haben. Ein weiteres Verfahren ist der sogenannte methanol to gasoline-Prozeß gemäß US-PS 3 928 483. In diesem bekannten Verfahren werden die aus Methanol und/oder Dimethylether an Aluminosilikatzeolith-Katalysatoren erhaltenen gasförmigen Reaktionsprodukte von den Flüssiganteilen getrennt und im Kreislauf gefahren. Die flüssigen Reaktionsprodukte enthalten Aromaten in begrenzten Mengen.

Bei diesem Verfahren erhält man ein Reaktionsgemisch, in dem neben etwa $1/3$ Aromatenanteilen noch gesättigte und ungesättigte aliphatische und naphthenische Kohlenwasserstoffe mit einer C-Zahl über 5 enthalten sind.

Es wurde nun gefunden, daß man eine höhere Ausbeute an wertvollen einkernigen Aromaten erhält, wenn man die Umsetzung in Gegenwart von mit Boehmit verstrangten Borosilikatzeolithen vom Pentasiltyp in einem Rückführverfahren ausführt, bei dem aus dem Umsetzungsgemisch bei einmaligem Durchgang durch die Katalysatorzone alle nicht aromatischen Anteile abgetrennt und eneut in die Katalysatorzone zurückgeführt und dort zusammen mit dem frisch zugesetzten Methanol und/oder Dimethylether umgesetzt werden.

Ein besonderer Vorteil des erfindungsgemäßen Verfahrens besteht darin, daß sich die Aromatenfraktion nahezu vollständig aus einkernigen Aromaten zusammensetzt. Die Endprodukte der Reaktion sind Methan und Aromaten.

Das in der Zeichnung wiedergegebene Verfahrensschema verdeutlich den grundlegenden Verfahrensablauf.

Aus Leitung (1) gelangen die Ausgangsstoffe Methanol und/oder Dimethylether in den Reaktor (2), in dem die Umwandlung in ein Kohlenwasserstoffgemisch an einem Zeolithkatalysator durchgeführt wird. Die Reaktionsprodukte, die $C_2$—$C_4$-Olefine, $C_1$—$C_4$-Paraffine, $C_5{}^+$-Aliphaten, Aromaten und Wasser enthalten, gelangen durch Leitung (3) in die Aufarbeitungsstufe (4). In dieser Aufarbeitungsstufe (4) werden die Reaktionsprodukte getrennt. Nach Abtrennung der Aromaten und des Wassers über Produktleitung (5) werden die übrigen Reaktionsprodukte über Leitung (6) in Reaktor (2) zurückgeführt, in dem sie erneut mit frisch zugeführtem Methanol und/oder Dimethylether umgesetzt werden.

Die Umsetzung erfolgt bei Temperaturen zwischen 300 und 650°C und bei einer Belastung WHSV (g eingesetztes $CH_3OH$ bzw. $CH_2$/g Katalysator und Stunde) von 0,2 bis 25 $h^{-1}$. Es kann wasserverdünntes Methanol, bevorzugt Rohmethanol z. B. mit 17 Gew.-% Wassergehalt verwendet werden. Das Rückführungsverhältnis Kohlenwasserstoff zu frisch zugesetztem Methanol kann zwischen 10 und 90 Gew.-% eingestellt werden.

Das nachfolgende Beispiel erläuterte die Durchführung des erfindungsgemäßen Verfahrens näher.

### Beispiel

Ein Borosilikatzeolith wird durch hydrothermale Synthese aus 66,1 g $SiO_2$ (Aerosil 200), 30,85 g $H_3BO_3$ in 881 g einer wäßrigen 1,3 Propandiamin-Lösung (Mischung 50 : 50) gefällt bzw. kristallisiert, bei 170°C 24 h getrocknet und bei 500°C 24 h calciniert. Dieser Borosilikatzeolith setzt sich zusammen aus 91,2% $SiO_2$ und 4,74% $B_2O_3$. Nach Verstrangung des Zeoliths mit Boehmit im Verhältnis 60 : 40 erhält man den verwendeten Katalysator.

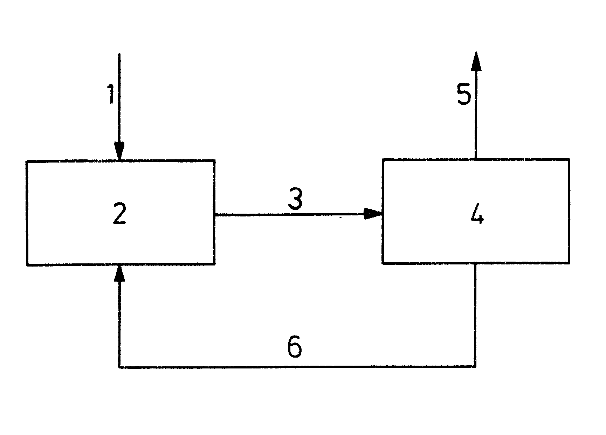

An dem oben beschriebenen Katalysator wird in einer Anlage, die in Fig. 1 wiedergegeben ist, bei 500°C Methanol zusammen mit den rückgeführten nichtaromatischen Verbindungen bei einer Belastung WHSV $H^{-1}$ = 4 bzw. 1,7 umgesetzt. Zum Vergleich wird dieselbe Umsetzung an einem Aluminiumsilikatzeolith-ZSM 5 nach US 3 928 483 ausgeführt. Die Ergebnisse werden in der Tabelle zusammengefaßt.

Das Einsatzgemisch setzt sich zusammen aus 50 Gew.-% Rohmethanol (17% Wassergehalt).

**0 090 284**

| | Katalysator nach Beispielen | | Katalysator nach US 3 928 483 |
|---|---|---|---|
| T° C | 500 | 500 | 400 |
| WHSV h⁻¹ | 4 | 1,7 | 2 |
| Gew.-% | | | |
| $C_1$ | 1,9 | 2,3 | 0,2 |
| $C_2$ | 2,5 | 6,8 | 1,2 |
| $C_3$ | 5,7 | 13,3 | 15,2 |
| $C_4$ | 3,1 | 8,2 | 25,9 |
| $C_5{}^+$-Aliphate | 28,9 | 11,3 | 21,2 |
| Aromaten | 57,9 | 58,1 | 37,0 |

**Patentanspruch**

Verfahren zur kontinuierlichen Herstellung von Aromaten durch Umsetzung von Methanol und/oder Dimethylether in Gegenwart von Zeolithen als Katalysatoren bei Temperaturen von 300 bis 650°C, dadurch gekennzeichnet, daß man die Umsetzung in Gegenwart von mit Boehmit verstrangten Borosilikatzeolithen vom Pentasiltyp in einem Rückführverfahren ausführt, bei dem aus dem Umsetzungsgemisch bei einmaligem Durchgang durch die Katalysatorzone alle nicht aromatischen Anteile abgetrennt und erneut in die Katalysatorzone zurückgeführt und dort zusammen mit dem frisch zugesetzten Methanol und/oder Dimethylester umgesetzt werden.

**Claim**

A process for the continuous production of aromatics by reacting methanol and/or dimethyl ether in the presence of a zeolite as catalyst at from 300 to 650°C, wherein the reaction is carried out, in the presence of a mixture of a borosilicate zeolite of the pentasil type with boehmite in the form of extrudates, in a recycle process in which all non-aromatic constituents are separated from the reaction mixture in a single pass through the catalyst zone and recycled to the catalyst zone where they are reacted together with the freshly added methanol and/or dimethyl ether.

**Revendication**

Procédé pour la préparation en continu d'hydrocarbures aromatiques par réaction de méthanol et/ou de diméthyléther en présence de zéolites servant de catalyseur et à des températures de 300 à 650°C, caractérisé en ce qu'on mène la réaction en présence de zéolites de borosilicate du type pentasil extrudées avec de la boehmite, en un procédé de recyclage dans lequel toutes les fractions non aromatiques sont séparées du mélange réactionnel en un seul passage à travers la zone du catalyseur et sont renvoyées dans la zone du catalyseur pour y être mises en réaction avec le méthanol et/ou le diméthyléther ajouté à l'état frais.

3